# EUROPÄISCHE PATENTSCHRIFT

(11) **EP 2 806 275 B1**
(45) Veröffentlichungstag und Bekanntmachung des Hinweises auf die Patenterteilung: **01.07.2015**
(21) Anmeldenummer: 13002699.0
(22) Anmeldetag: 23.05.2013
(51) Int. Cl.: G01N 33/68, C12Q 1/04, C12Q 1/18

(54) **Massenspektrometrische Resistenzbestimmung durch Wachstums-Messung**
Determination of resistance by means of growth measurement using mass spectrometry
Détermination de la résistance par spectrométrie de masse par mesure de croissance

(43) Veröffentlichungstag der Anmeldung: 26.11.2014
(73) Patentinhaber: Bruker Daltonik GmbH, 28359 Bremen (DE)
(72) Erfinder: Sparbier, Katrin, DE-28357 Bremen (DE); Lange, Christoph, DE-28879 Grasberg (DE)

(56) Entgegenhaltungen:
- WO-A1-2011/154517
- WO-A1-2012/016929
- WO-A1-2012/023845
- WO-A2-01/94910
- WO-A2-2005/031304
- DE-A1- 19 754 978
- DE-B4-102006 021 493
- US-A1- 2009 286 225
- SPARBIER K ET AL: "MALDI-TOF MS for functional detection of beta-lactam resistance of positive blood cultures", INTERNATIONAL JOURNAL OF MEDICAL MICROBIOLOGY; 63RD ANNUAL MEETING OF THE GERMAN-SOCIETY-FOR-HYGIENE-AND-MICROBIOLOG Y, URBAN UND FISCHER, DE; ESSEN, GERMANY, Bd. 301, Nr. Suppl. 1, 1. September 2011 (2011-09-01), Seite 1, XP008164831, ISSN: 1438-4221, DOI: 10.1016/J.IJMM.2011.08.002 [gefunden am 2011-08-26]
- SPARBIER K ET AL: "An automated evaluation algorithm for the MALDI-TOF MS based functional beta-lactamase assay", INTERNATIONAL JOURNAL OF MEDICAL MICROBIOLOGY; 64TH ANNUAL MEETING OF THE GERMAN-SOCIETY-FOR-HYGIENE-AND-MICROBIOLOG Y (DGHM), URBAN UND FISCHER, DE; HAMBURG, GERMANY, Bd. 302, Nr. Suppl. 1, 1. September 2012 (2012-09-01), Seite 7, XP008164830, ISSN: 1438-4221, DOI: 10.1016/J.IJMM.2012.08.001 [gefunden am 2012-09-10]
- GRIFFIN PM ET AL: "Use of Matrix-Assisted Laser Desorption Ionization -Time of Flight Mass Spectrometry to Identify Vancomycin-Resistant Enterococci and Investigate the Epidemiology of an Outbreak", JOURNAL OF CLINICAL MICROBIOLOGY, Bd. 50, Nr. 9, 27. Juni 2012 (2012-06-27), Seiten 2918-2931, XP002713214, DOI: 10.1128/JCM.01000-12
- DYTFELD DOMINIK ET AL: "Proteomic Profiling of Multiple Myeloma (MM) Cells Using iTRAQ and Label-Free Quantitative Proteomics for the Prediction of Complete or near Complete Response (CR/nCR) In Frontline Treatment with Lenalidomide, Bortezomib, and Dexamethasone", BLOOD; 52ND ANNUAL MEETING OF THE AMERICAN-SOCIETY-OF-HEMATOLOGY (ASH), AMERICAN SOCIETY OF HEMATOLOGY, US; ORLANDO, FL, USA, Bd. 116, Nr. 21, 19. November 2010 (2010-11-19), Seiten 271-272, XP008164832, ISSN: 0006-4971
- HIROAKI SATO ET AL: "Ribosomal protein profiling by matrix-assisted laser desorption/ionization time-of-flight mass spectrometry for phylogenety-based subspecies resolution of", SYSTEMATIC AND APPLIED MICROBIOLOGY, Bd. 34, Nr. 1, 1. Februar 2011 (2011-02-01), Seiten 76-80, XP028145455, ISSN: 0723-2020, DOI: 10.1016/J.SYAPM.2010.07.003 [gefunden am 2010-08-13]
- IGNAZIO GARAGUSO ET AL: "Matrix layer sample preparation: An improved MALDI-MS peptide analysis method for proteomic studies", PROTEOMICS, Bd. 8, Nr. 13, 1. Juli 2008 (2008-07-01), Seiten 2583-2595, XP55086237, ISSN: 1615-9853, DOI: 10.1002/pmic.200701147
- GUSTAFSSON JOR ET AL: "Internal calibrants allow high accuracy peptide matching between MALDI imaging MS and LC-MS/MS", JOURNAL OF PROTEOMICS, Bd. 75, 23. Mai 2012 (2012-05-23), Seiten 5093-5105, XP002715751,

## Beschreibung

Die Erfindung betrifft ein massenspektrometrisches Verfahren zur Bestimmung der mikrobiellen Resistenzen gegen Antibiotika.

### Definitionen

In dieser Schrift wird statt der gesetzlichen "vereinheitlichten atomaren Masseneinheit" (u) die Einheit "Dalton" (Da) verwendet, die in der letzten (achten) Ausgabe 2006 der Schrift "The International System of Units (SI)" des "Bureau International des Poids et Mesures" der atomaren Masseneinheit gleichwertig beigestellt wurde; vor allem, wie dort angemerkt, um die Einheiten Kilodalton, Millidalton und Ähnliche verwenden zu können.

Aus Gründen der Vereinfachung wird in dieser Schrift nur der Begriff "Proteine" verwendet, obwohl im bevorzugten Massenbereich von 3000 bis 15000 Dalton die Proteine oft besser als "Peptide" zu bezeichnen wären. Der Übergang der leichteren Peptide zu den schwereren Proteinen ist fließend und nicht eindeutig definiert.

Als Abkürzung für "Mikroorganismen" wird hier häufig der Begriff der "Mikroben" verwendet. Unter dem Singular "Mikrobe" wird, wie im allgemeinen Sprachgebrauch üblich, neben einer einzelnen Mikrobenzelle auch die Mikrobenart verstanden. Unter dem Plural "Mikroben" werden die der Untersuchung unterliegenden Mikrobenzellen verstanden.

### Stand der Technik

Viele Arten von Mikroorganismen, insbesondere Bakterien und einzellige Pilze wie Hefen, lassen sich heutzutage schnell und mit geringen Fehlerraten massenspektrometrisch identifizieren. Die Identifizierung erfolgt im Routinebetrieb durch die Berechnung von Ähnlichkeitswerten zwischen einem Massenspektrum der zellaufgeschlossenen Mikroben, insbesondere deren lösliche Proteine, und gleichartigen Referenzmassenspektren von bekannten Mikroorganismen. Überschreiten die Ähnlichkeitswerte bestimmte Grenzwerte, so können Familie, Gattung, Spezies oder sogar Stamm identifiziert werden. Dieses sehr schnelle und preiswerte Identifizierungsverfahren für Mikroorganismen hat sich sowohl in groß angelegten Studien wie auch im täglichen Einsatz in vielen mikrobiologischen Laboratorien als außerordentlich erfolgreich erwiesen. Je nach Gerät können 48 bis 384 Mikrobenproben zeitparallel bestimmt werden; ab dem Ende der Aufzucht einer Kolonie dauert es nur Minuten bis zur Identifizierung. Das Verfahren hat sehr geringe Fehlerraten, weit geringer als die Fehlerrate herkömmlicher mikrobiologischer Identifizierungsverfahren, selbst geringer als die der DNA-Analysen. Es gibt inzwischen Massenspektrometer, zugehörige Auswerteprogramme und Bibliotheken mit Referenzspektren auf dem Markt, die nach Medizinproduktegesetz und anderen, nationalen oder internationalen Vorschriften und Richtlinien als IVD-Produkte für medizinische Diagnostik zugelassen sind.

Der Begriff "Antibiotikum" bezeichnet, wie im allgemeinen Sprachgebrauch üblich, einen pharmakologischen Wirkstoff für die Behandlung mikrobieller Infektionskrankheiten als und auch Substanzen zur Desinfektion. Die Erfolge des Penicillins führten zur Suche und Entdeckung vieler weiterer Antibiotika. Es gibt Breitband-Antibiotika, die gegen viele Mikrobenfamilien wirksam sind, und schmalbandige Antibiotika, die gezielt gegen einzelne Mikrobenarten wirken.

Seit Penicillin als erster pharmakologischer Wirkstoff eingesetzt wurde, haben Mikrobenstämme in zunehmendem Maße verschiedenartige Resistenzen gegen verschiedenartige Antibiotika entwickelt oder von anderen Mikroben übernommen, sich also Eigenschaften angeeignet, die es ihnen ermöglichen, die Wirkung von antibiotisch wirkenden Substanzen abzuschwächen oder ganz zu neutralisieren. Resistenzen sind inzwischen leider häufig; in Krankenhäusern vorkommende Mikroben sind heute überwiegend resistent. In einigen Fällen kommt es vor, dass die Resistenz einer im Krankenhaus übertragenen Mikrobe gegen die im Krankenhaus üblichen Antibiotika vorhergesagt werden kann; für Infektionen, die außerhalb des Krankenhauses erworben wurden, gilt das aber nicht.

Der Erfolg einer Therapie bei mikrobiellen Infektionen, die in Akutsituationen wie einer Sepsis, oder als Sekundärinfektion bei einer schon vorhandenen Primärerkrankung (oder Primärinfektion) meist lebensbedrohlich sind, hängt oft davon ab, dass schon die erste Verabreichung eines Antibiotikums wirksam ist. Eine gezielte Verabreichung setzt nicht nur eine möglichst schnelle und fehlerfreie Identifizierung des Krankheitserregers voraus, sondern auch eine möglichst schnelle Bestimmung von dessen Resistenz gegen verschiedene Antibiotika. Die klassische Bestimmung der Resistenz besteht in einer Kultivierung in Anwesenheit eines Antibiotikums, ist aber leider sehr langwierig: sie dauert 24 bis 48 Stunden.

Neben der Kultivierung bei Anwesenheit von Antibiotika gibt es auch genetische Verfahren zur Bestimmung von Resistenzen. Der Nachweis einer Resistenz erfolgt dabei durch den Nachweis von bekannten Resistenzgenen im Genom des jeweiligen Krankheitserregers. Ein Vorteil der genetischen Verfahren besteht darin, dass die Resistenzgene durch Techniken wie der Polymerasen-Kettenreaktion (PCR) vervielfältigt werden können und damit die Analysedauer nicht mehr durch die Vermehrungsrate der Bakterien bestimmt wird. Die Nachteile bestehen darin, dass sie im Vergleich zum Routineverfahren mit höheren Kosten verbunden sind und keine funktionellen Tests darstellen. So kann zwar ein Resistenzgen vorhanden sein, aber nicht exprimiert werden, wodurch der zu untersuchende Bakterienstamm nicht resistent ist, aber durch das Verfahren als resistent erkannt wird.

Aus der Patentschrift DE 10 2006 021 493 B4 (V. M. Govorun und J. Franzen, 2006, entsprechend GB 2438066 B, US 8,293,496 B2; im Folgenden als "Govorun" bezeichnet) sind massenspektrometrische Verfahren zur Resistenzbestimmung von Mikroben bekannt.

In einer Ausführungsform werden beispielsweise Proteinprofile der Mikroben nach Kultivierung in Medien mit und ohne Zugabe von Antibiotika massenspektrometrisch gemessen und verglichen werden. Dabei werden die Mikroben beispielsweise in Zentrifugenröhrchen mit und ohne Antibiotika kultiviert. Die Mikroben werden nach der Kultivierung auszentrifugiert, gewaschen, und dann mit Säuren und Acetonitril in dne Zentrifugenröhren so aufgeschlossen, dass ihre löslichen Proteine frei werden. Ein geringe Menge (etwa ein Mikroliter) dieser Aufschlussflüssigkeit wird auf den Probenträger präpariert, getrocknet und dann mit einer geringen Menge an Matrixlösung (ebenfalls etwa ein Mikroliter) überschichtet. Die gelösten Proteine werden in die Matrixkristalle eingebaut, die beim Trocknen entstehen. Die Proben mit Matrixkristallen und eingebauten Proteinen werden im Massenspektrometer mit Laserlichtpulsen beschossen, wodurch sich einem Verdampfungsplasma Ionen der Proteinmoleküle bilden. Die Messung ihrer Flugzeit in einem Flugzeitmassenspektrometer ergibt das Massenspektrum der Mikrobe, das im Wesentlichen aus den Proteinpeaks besteht.

Aus der Ähnlichkeit der beiden Massenspektren kann nur bedingt nicht auf die Resistenz der Mikrobe geschlossen werden. Werden die suszeptiblen Mikroben nur wachstumsgehemmt oder abgetötet, ohne zerlegt zu werden, wie beispielsweise Klebsiellen aus der Familie der Enterobacteriaceae, so ergeben sie ein Massenspektrum, das denen der Mikroben aus Medien ohne Antibiotikum praktisch gleicht. Der Grund dafür ist, dass die Ausbildung der Massenspektren, die mit einer Ionisierung durch matrixunterstützte Laserdesorption (MALDI) gewonnen werden, nur geringfügig von der Menge der Mikroben in der Probe abhängt. Probenpräparationen mit Zehntausend Mikroben liefern praktisch die gleichen Massenspektren wie Probenpräparationen mit zehn Millionen Mikroben, was ideal für eine Identifizierung ist, aber nicht für die Erkennung der Resistenz. Werden die Mikroben nur im Wachstum gestoppt, so ergeben sie die gleichen Massenspektren, weil nur ein mengenmäßiger Unterschied besteht, der aber in den Massenspektren nicht sichtbar wird.

Wie im Patent Govorun weiter ausgeführt wird, kann die Resistenz auch dadurch erkannt werden, dass man den zu untersuchenden Mikroben eine zweite Mikrobensorte hinzufügt, die ein stark unterschiedliches Massenspektrum liefert, resistent ist und auf jeden Fall bei Anwesenheit des Antibiotikums weiter wächst. Das Massenspektrum mit den überlagerten Proteinen beider Mikroben sollte dann die Unterschiede im Wachstum zeigen. Leider hat sich dieses Verfahren in der Routine aus verschiedenen Gründen als nicht sehr praktikabel erwiesen; es setzt zumindest grobe Mengenbestimmungen der eingesetzten Mikroben voraus, ungefähr gleiche Wachstumsgeschwindigkeiten in dem verwendeten Kultivierungsmedium, und Resistenz der Referenzmikroben gegen viele Antibiotika.

Die Mikroben, deren Resistenz festgestellt werden soll, liegen bevorzugt in genügender Menge in genügend reiner Form vor. Sie können beispielsweise Kolonien auf einem Agar bilden, oder aber als Mikroben aus einer Blutkultur vorliegen. Bei Agar-Kulturen ist es dabei gängige Praxis, die Mikroben nicht nur einer Kolonie für die Prüfung zu verwenden, sondern die Mikroben aus mindestens fünf Kolonien gemeinsam dieser Prüfung zu unterziehen, um gegebenenfalls auch die Anwesenheit einer resistenten Mikrobe unter nichtresistenten Mikroben der gleichen Art erkennen zu können. Einer geschulten und erfahrenen Fachkraft ist es im Allgemeinen möglich, Kolonien gleichartiger Mikroben zu erkennen und diese zu sammeln. Sie sind dann zu mischen und für die Kulturen zu teilen. Bei Mikroben aus Blutkulturen ist eine Mischung aus den verschiedenen Mikroben, die bei der Infektion übertragen wurden, von Natur aus gegeben.

Einer Resistenzbestimmung (auch einer solchen nach Govorun) geht in der Regel oft die Identifizierung der Mikroben voraus, die auf der Agar-Kultur oder in der Blutkultur gewachsen sind. Dabei ist es hilfreich, besonders für das Verfahren nach Govorun, die Mikrobenart und deren Wachstumsgeschwindigkeit zu kennen. Auch sind gewöhnlich für diese Mikrobe die Antibiotika bekannt, gegen die sie resistent sein kann. Des Weiteren sind für gewöhnlich auch die minimalen Hemmkonzentrationen (MHK) für suszeptible Mikroben bekannt. Dadurch können Kulturen mit diesen Antibiotika in geeigneten Konzentrationen angesetzt werden; aus den bekannten Wachstumsgeschwindigkeiten ergeben sich die minimalen Kultivierungsdauern für das Govorun-Verfahren.

### Aufgabe der Erfindung

Es ist die Aufgabe der Erfindung, ein massenspektrometrisches Verfahren bereitzustellen, mit dem eine Resistenz von Mikroben gegenüber einem oder auch mehreren Antibiotika sicher, preiswert, weitgehend automatisierbar und vor allem schnell bestimmt werden kann, insbesondere für schnell wachsende und damit besonders gefährliche Keime möglichst in nur einer Stunde. Das Verfahren soll dabei möglichst mit den gleichen Routine-Massenspektrometern durchgeführt werden können, die auch für die massenspektrometrische Identifizierungen der Mikroben benutzt werden.

### Kurze Beschreibung der Erfindung

Die Erfindung stellt ein Verfahren zur massenspektrometrischen Bestimmung der Resistenz von Mikroben, in dem die Mikroben in einem Medium mit einem Antibiotikum kultiviert werden und ein Massenspektrum MS_{cum} der Mikroben nach deren Kultivierung aufgenommen wird. Das erfindungsgemäße Verfahren ist dadurch gekennzeichnet, dass der während der Kultivierung gegebenenfalls stattfindende Zuwachs an Mikroben anhand einer dosiert zugegebenen, im Massenspektrum MS_{cum} mitgemessenen Referenzsubstanz bestimmt wird, wobei ein Zuwachs an Mikroben die Resistenz gegen das Antibiotikum anzeigt. Ein Zuwachs an Mikroben kann dadurch bestimmt werden, dass die Mikrobenmenge nach der Kultivierung massenspektrometrisch ermittelt wird und mit einer entsprechend ermittelten Mikrobenmenge vor der Kultivierung und/oder mit einer entsprechend ermittelten Mikrobenmenge nach einer Kultivierung ohne das Antibiotikum verglichen wird.

Eine erste bevorzugte Ausführungsform besteht darin, dass die Mikrobenmenge nach der Kultivierung aus dem Massenspektrum MS_{cum} ermittelt wird und dass die Mikroben als resistent ausgewiesen werden, wenn die Mikrobenmenge größer als ein vorgegebener Grenzwert ist. Dabei ist es vorteilhaft, dass die Mikrobenmenge zu Beginn der Kultivierung standardisiert ist.

Eine zweite bevorzugte Ausführungsform besteht darin, die Mikroben zusätzlich in dem Medium ohne das Antibiotikum kultiviert werden, dass ein Massenspektrum MSₛᵢₙₑ der Mikroben nach deren Kultivierung ohne Antibiotikum aufgenommen wird, in dem die dosiert zugegebene Referenzsubstanz mitgemessen wird, und dass die Mikroben als resistent ausgewiesen werden, wenn sich die aus den Massenspektren MS_{cum} und MSₛᵢₙₑ ermittelten Mikrobenmengen relativ oder absolut um weniger als einen vorgegebenen Grenzwert unterscheiden.

Eine dritte bevorzugte Ausführungsform besteht darin, dass vor der Kultivierung zusätzlich ein Massenspektrum MS₀ der Mikroben aufgenommen wird, in dem die dosiert zugegebene Referenzsubstanz mitgemessen wird, und dass die Mikroben als resistent ausgewiesen werden, wenn die aus dem Massenspektrum MS_{cum} ermittelte Mikrobenmenge signifikant größer als die aus dem Massenspektrum MS₀ ermittelte Mikrobenmenge ist. In dieser Ausführungsform können dabei die Mikroben zu dem Medium gegeben werden, das danach in zwei (bevorzugt gleich große) Kulturen aufgeteilt wird, die für Kultivierung mit dem Antibiotikum bzw. für die Aufnahme des Massenspektrums MS₀ verwendet werden. Andererseits kann auch ein Teil des Mediums nach oder zeitnah zur Zugabe des Antibiotikums entnommen und für die Aufnahme des Massenspektrums MS₀ verwendet werden.

Die in den drei bevorzugten Ausführungsformen verwendeten Entscheidungskriterien können gegebenenfalls auch durch logischen Operatioren, wie z.B. eine Konjunktion oder eine nicht-ausschließende Disjunktion, kombiniert werden.

Der Begriff "Massenspektrum" umfasst die massenspektrometrischen Rohdaten bis hin zu einer bearbeiteten Peakliste, die nur noch die Positionen und Intensitäten von Massensignalen enthält. Dabei kann ein Massenspektrum aus einer Vielzahl von Intensitätswerten in einem zusammenhängenden Massenbereich als auch aus den Intensitätswerten mehrerer getrennter Massenbereichen bestehen. Das Massenspektrum kann vor der Ermittlung der Mikrobenmenge einer Signalverarbeitung unterzogen werden, die beispielsweise eine Korrektur (Subtraktion) der Basislinie, eine Glättung von Massensignalen, eine Eliminierung von Rauschsignalen und/oder eine Auswahl von Massensignalen über einem festgelegten Rauschwert umfasst.

Die Mikrobenmenge oder ein Maß dafür kann aus einem Massenspektrum auf unterschiedliche Arten ermittelt werden, beispielsweise durch den Quotienten aus der Intensität eines Mikrobensignals und der Intensität eines Referenzsignals oder den summierten Intensitäten mehrerer Referenzsignale, den Quotient aus den summierten Intensitäten mehrerer Mikrobensignale und der Intensität eines Referenzsignals oder den summierten Intensitäten mehrerer Referenzsignale, oder den Quotient aus den summierten Intensitäten aller Signale eines Teiles oder des ganzen Massenspektrums und der Intensität eines Referenzsignals oder den summierten Intensitäten mehrere Referenzsignale. Vor der Summation können Mikrobensignale aus einer Peakliste oder einem kontinuierlichen Massenspektrum ausgewählt werden, beispielsweise solche mit einer großen Häufigkeit in Wiederholungsmessungen oder solche mit einer großen Signalintensitäten. Neben Peaklisten können auch die massenspektrometrischen Rohdaten (selbst ohne eine kalibrierte Massenachse, wenn die Positionen der Referenzsubstanzen bekannt sind) zur Ermittlung der Mikrobenmenge verwendet werden, indem beispielsweise alle Intensitätswerte in einem ausgewählten Bereich der Rohdaten summiert werden und durch die summierten Intensitätswerte in einem Bereich mit einem Referenzsignal geteilt wird, wobei gegebenenfalls vorher die Basislinie korrigiert wird.

Die Mikroben können in dem Medium unter Zugabe eines einzelnen Antibiotikums, aber auch unter Zugabe eines Gemisch verschiedener Antibiotika kultiviert werden. Um die Stärke der Resistenz zu bestimmen oder zumindest abschätzen zu können, können die Mikroben gleichzeitig in mehreren Kulturen mit jeweils einer anderen Konzentration eines Antibiotikums kultiviert werden, wobei für jede Kultur ein Massenspektrum MS_{cum,i} der Mikroben und der dosiert zugegebenen Referenzsubstanz aufgenommen wird. Für die Prüfung der Resistenz gegenüber mehreren Antibiotika können zeitgleich mehrere Kulturen mit mehreren Antibiotika angesetzt werden, erforderlichenfalls sogar mit jeweils verschiedenen Konzentrationsabstufungen der Antibiotika, wobei wiederum für jede Kultur ein Massenspektrum MS_{cum,i} der Mikroben und der dosiert zugegebenen Referenzsubstanz aufgenommen wird.

Die Referenzsubstanz kann dem Medium vor, während und nach der Kultivierung, während der Präparation einer massenspektrometrischen Probe oder während der Aufnahme des Massenspektrums dosiert zugegeben werden. Die Referenzsubstanz kann nach dem Zellaufschluss der Mikroben dosiert zugegeben werden. Wird die Referenzsubstanz dem Medium zugegeben, darf sie nicht von den Mikroben aufgenommen oder abgebaut werden; bei einer Zugabe nach Zellaufschluss entfällt diese Bedingung im Wesentlichen. Werden die Massenspektren mittels Ionisierung durch matrixunterstützte Laserdesorption (MALDI) aufgenommen, wird die Referenzsubstanz bevorzugt erst bei der Präparation der MALDI-Proben auf einem Probenträger zugegeben, insbesondere zusammen mit der Matrixlösung.

Es kann auch ein Gemisch von Referenzsubstanzen dosiert zugegeben werden. Dabei ist es günstig, wenn ein größerer Konzentrationsbereich durch die Referenzsubstanzen im Gemisch abgedeckt wird, so können beispielsweise drei Referenzsubstanzen in Verhältnissen von 100:10:1 oder 25:5:1 verwendet werden. Eine Referenzsubstanz soll gut zu ionisieren sein und nach Möglichkeit mehrere erkennbare Referenzsignale in den entsprechenden Massenspektren liefern. Die Protonenaffinität der Referenzsubstanz ist dabei bevorzugt größer als die Protonenaffinität der Mehrzahl der Mikrobenproteine. Bevorzugte Referenzsubstanzen sind Ribonukleasen oder Lysozyme. Die Masse der Referenzsubstanzen liegt bevorzugt zwischen 10 und 20 Kilodalton, insbesondere zwischen 14 und 15 Kilodalton.

Die zu untersuchenden Mikroben liegen bevorzugt in hinreichend reiner Form vor. Sie können beispielsweise Kolonien auf einem Agar bilden, aber auch aus einer Blutkultur gewonnen werden. Es ist auch möglich, dass Mikroben einer zu untersuchende Probe, z.B. der Abstrich einer Nasenschleimhaut, direkt oder nach einer Vorvermehrung in einem Flüssigmedium mit dem erfindungsgemäßen Verfahren untersucht werden. Für die Durchführung des erfindungsgemäßen Verfahrens ist es prinzipiell nicht notwendig, dass die Mikrobenmenge vor oder nach einer Kultivierung mit bloßem Auge sichtbar ist, d.h., dass vor der Kultivierung gegebenenfalls weniger als 10⁵ Mikrobenzellen, insbesondere weniger als 10⁴ Mikrobenzellen, ausreichen, wobei die Kultivierungen bevorzugt in Volumina zwischen 1µl und 1ml, insbesondere in 100µl, durchgeführt werden. Eine geringe Ausgangsanzahl von Mikrobenzellen in einer zu untersuchenden Probe und eine kurze Kultivierungszeit können es erforderlich machen, die Proteine der Mikroben vor oder im Zuge der Präparation einer massenspektrometrischen Probe aufzukonzentrieren. Das Aufkonzentrieren kann dabei beispielsweise durch das Ausfällen der Proteine nach einem Zellaufschluss der Mikroben mit anschließender Separation der Proteine, z.B. durch Zentrifugieren, oder mit Hilfe von für die Proteinbindung funktionalisierten Oberflächen erfolgen, wie sie etwa auf (magnetischen) Beads, auf MALDI-Probenträgern oder auf der Oberfläche des Füllmaterials von Pipettenspitzen vorhanden sein können. Separierte und mit Proteinen beladene Beads können gegebenenfalls direkt auf einen MALDI-Probenträger aufgebracht werden.

Das erfindungsgemäße Verfahren kann für die Bestimmung der Resistenz von Bakterien als auch für die Bestimmung der Resistenz von einzelligen Pilzen, wie Hefen, auf ein Antimykotikum oder auf ein Gemisch von Antimykotika verwendet werden. Eine weitere Ausführungsform des erfindungsgemäßen Verfahrens besteht darin, dass die Mikroben vor der Bestimmung der Resistenz taxonomisch identifiziert werden und dass die Referenzsubstanz, Grenzwerte zur Festlegung der Resistenz, das Medium und/oder die Kultivierungsbedingungen, insbesondere die Kultivierungszeit, aufgrund der taxonomischen Einordnung ausgewählt werden.

Die Erfindung ähnelt in einer Ausführungsform dem Verfahren von Govorun, zielt aber darauf, das Mikrobenwachstum insbesondere durch die Proteinzuwächse messen zu können. Um den Zuwachs an Mikroben in den Medien mit Antibiotika anhand ihrer Massenspektren quantitativ bestimmen zu können, werden der Kultur oder dem Zellaufschluss eine oder mehrere geeignete, genau dosierte Referenzsubstanzen zugesetzt. Es wird also die Vermehrung der Biomasse, und damit insbesondere der Proteine, in den Medien mit Antibiotika anhand der Referenzsubstanzen bestimmt. Es kann dabei ein Vergleich mit den Mikrobenzuwächsen in Medien ohne Antibiotika vorgenommen werden, aber auch ein Vergleich mit der Mikrobenmenge ohne weitere Kultivierung. Ein Zuwachs der Biomasse in erwartetem Maße zeigt die Resistenz der untersuchten Mikroben gegen das Antibiotikum in der verwendeten Konzentration an; suszeptible Mikroben zeigen kein messbares Wachstum, wenn die Konzentration des Antibiotikums über der minimalen Hemmkonzentration (MHK) liegt.

Das Verfahren hat sich als sehr schnell erwiesen: Da nur etwa zwei Verdopplungsgenerationen notwendig sind, um die Biomasse um einen Faktor vier wachsen zu lassen, kann für gefährliche Infektionen, die in der Regel auf schnell wachsenden Mikroben mit 20 Minuten Verdopplungszeit beruhen, bereits nach einer Kultivierungsdauer von 40 Minuten die Resistenz nachgewiesen werden; für langsamer wachsende Mikroben sind längere Zeiten erforderlich. Die benötigte Kultivierungsdauer kann durch die Kenntnis der Mikrobenart und damit Kenntnis ihrer Verdoppelungszeit festgelegt werden.

### Kurze Beschreibung der Abbildungen

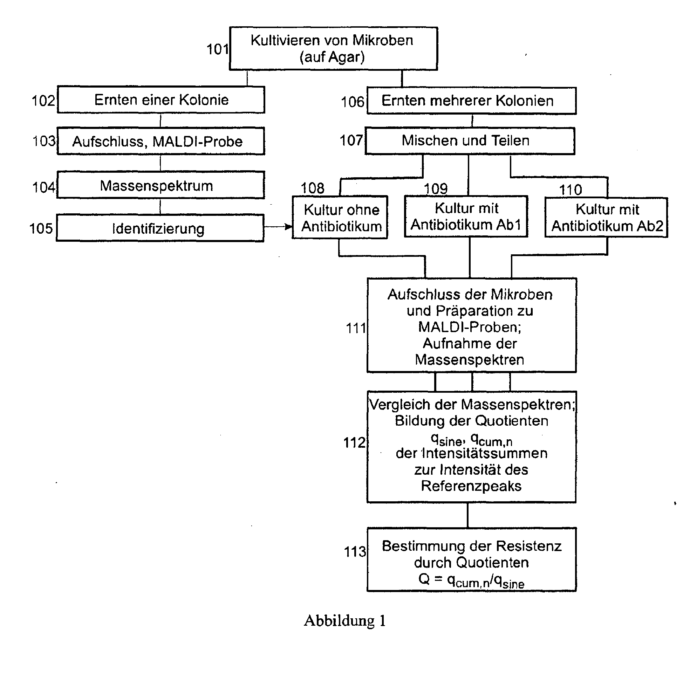

Abbildung 1 zeigt ein Beispiel eines Ablaufdiagramms für ein bevorzugtes Verfahren zur Identifizierung einer Mikrobe und zur Bestimmung von deren Resistenz nach dieser Erfindung.

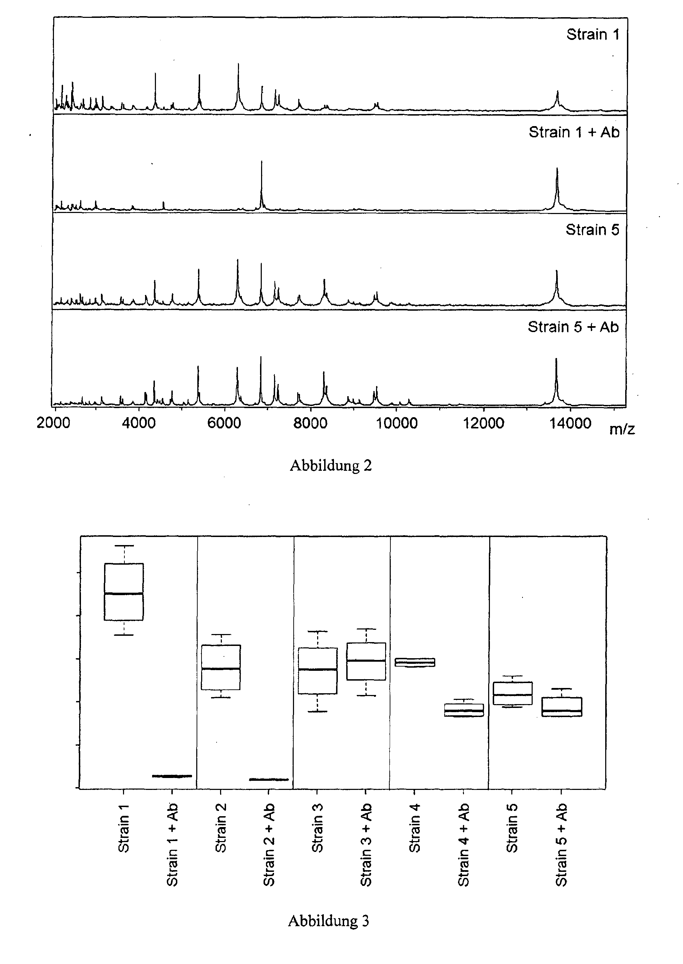

Abbildung 2 gibt vier Massenspektren zweier Stämme einer Art von Bakterien aus der Gattung Klebsiellen wieder: Die beiden oberen Massenspektren stammen vom Stamm 1 ("strain 1"), der suszeptibel ist, so dass in der Kultur mit Zugabe des Antibiotikums ("Ab") kein Wachstum stattgefunden hat. In diesem zweiten Spektrum ("strain 1 + Ab") von oben sind praktisch nur noch die Massenpeaks der Referenzsubstanz (Ribonuklease A) sichtbar, ganz rechts einfach geladen, leicht links von der Mitte doppelt geladen. Die beiden unteren Massenspektren sind vom Stamm 5 gewonnen, der resistent ist, so dass auch bei Zugabe des Antibiotikums ("strain 5 + Ab") ungestörtes Wachstum vorliegt (unterstes Spektrum). Alle Massenspektren wurden nach einer Kultivierungsdauer von nur einer Stunde aufgenommen.

Abbildung 3 zeigt eine Auswertung der Massenspektren von fünf Klebsiella-Stämmen, jeweils ohne und mit Antibiotikum. Stamm1 und Stamm 2 sind suszeptibel, daher kein Wachstum bei Antibiotikum. Stamm 3 und 5 sind resistent, gleiches Wachstum ohne und mit Antibiotikum. Für Stamm 4 kann angenommen werden, dass entweder eine intermediäre Resistenz vorliegt, oder dass nur ein Teil der eingesammelten Kolonien resistent ist, so dass die Ausgangsmenge an resistenten Mikroben kleiner war. Die Kästen geben die mittleren Streuungen bei Wiederholungsmessungen wieder.

### Bevorzugte Ausführungsbeispiele

Die Erfindung schlägt ein bevorzugtes Verfahren zur massenspektrometrischen Bestimmung der Resistenz von Mikroben durch Vergleich der Massenspektren der Mikroben nach deren Kultivierung in mehreren gleichartigen Medien mit und ohne Zugabe von Antibiotika vor, das dadurch gekennzeichnet ist, dass der Zuwachs an Mikroben während der Kultivierung anhand mindestens einer zugegebenen Referenzsubstanz bestimmt wird, wobei ein Zuwachs an Mikroben in einem Medium mit Antibiotikum die Resistenz gegen dieses Antibiotikum in der gegebenen Konzentration anzeigt. Die Mikroben können insbesondere auch gleichzeitig in mehreren Medien mit verschiedenartigen Antibiotika kultiviert werden.

Die Referenzsubstanz kann bereits dem Kultivierungsmedium dosiert zugegeben werden, muss aber dann so beschaffen sein, dass sie von den Mikroben in stets gleicher Menge aufgenommen und nicht abgebaut wird, um nach Waschen und Zellaufschluss in der Präparation sichtbar zu sein. Da diese Bedingung schwer zu erfüllen ist, wird die Referenzsubstanz bevorzugt erst nach der Kultivierung zugegeben, beispielsweise dem Zellaufschluss oder, im Falle einer Ionisierung durch matrixunterstützte Laserdesorption, der Präparation der Probe auf dem Probenträger.

Um die Stärke der Resistenz festzustellen, oder um festzustellen, ob die Mikroben aller eingesammelten Kolonien in gleicher Weise resistent sind oder nicht, können die Mikroben in Medien mit Antibiotika in mehreren Konzentrationsabstufungen kultiviert werden. Ist nur ein Teil der gesammelten Kolonien resistent, wofür hier die Bezeichnung "Mischresistenz" gebraucht wird, so ist für alle Konzentrationsstufen ein gleich proportioniert geringeres Wachstum als für die Kultur ohne Antibiotikum zu sehen, weil das Wachstum mit einer geringeren Menge an resistenten Mikroben gestartet wurde. Dieser Fall ist jedoch relativ selten. Bei intermediärer Resistenz dagegen findet bei hohen Konzentrationen des Antibiotikums kein Wachstum mehr statt, bei geringer Konzentration dagegen volles Wachstum. Es ist durchaus auch möglich, die Mikroben der Kolonien nicht zu mischen, sondern für die Mikroben jeder Kolonie diese Tests jeweils einzeln durchzuführen.

Für die Identifizierung werden Mikroben in der bisherigen Praxis auf einem Agar oder in einer Blutkultur kultiviert, meist über Nacht. Bei Agar-Kulturen liegen dann in der Regel mehrere Kolonien vor, von denen eine zur Identifizierung geerntet wird. Für die Bestimmung der Resistenz ist es gängige Praxis, die Mikroben aus mindestens fünf weiteren Kolonien gemeinsam dieser Prüfung zu unterziehen, um gegebenenfalls auch die Anwesenheit einer resistenten Mikrobenkolonie unter nichtresistenten Mikrobenkolonien der gleichen Art erkennen zu können (Mischresistenz). Einer geschulten und erfahrenen Fachkraft ist es im Allgemeinen möglich, Kolonien gleicher Mikroben zu erkennen und diese zu sammeln. Die Mikroben dieser Kolonien können dann bevorzugt gemischt und auf die verschiedenen Kulturen aufgeteilt werden, sie können aber auch in Sonderfällen alle einzeln der Resistenzbestimmung unterworfen werden, beispielsweise dann, wenn für die Kolonien nicht mit Sicherheit davon ausgegangen werden kann, dass sie der gleichen Mikrobenart angehören. Bei Mikroben aus Blutkulturen ist eine Mischung aus den verschiedenen Mikroben, die bei der Infektion übertragen wurden, von Natur aus gegeben. Die Blutkultur endet, wie dem Fachmann bekannt, meist in einem Zentrifugen-Pellet, das genügend Mikroben für die Identifizierung und auch für die Resistenzbestimmung enthält.

Die Erfindung ähnelt in einer Ausführungsform dem Verfahren von Govorun, in dem Massenspektren der Mikroben in Kulturen mit und ohne Zusatz von Antibiotika miteinander verglichen werden, ist aber auf die Messung des Mikrobenzuwachses ausgerichtet, die bei Govorun nicht erwähnt wird. Um den Zuwachs an Mikroben in den Medien mit Antibiotika anhand ihrer Massenspektren quantitativ bestimmen zu können, werden eine oder mehrere geeignete, genau dosierte Referenzsubstanzen zugesetzt, bevorzugt nach Zellaufschluss. Es wird also die Vermehrung der Biomasse, und damit einhergehend insbesondere auch der Proteine, in den Medien mit Antibiotika anhand der Referenzsubstanzen quantitativ bestimmt. Es kann dabei insbesondere ein Vergleich mit den Mikrobenzuwächsen in Medien ohne Antibiotika vorgenommen werden, aber auch ein Vergleich mit der Mikrobenmenge der eingesetzten Mikroben ohne weitere Kultivierung. Eine Vermehrung der Biomasse in erwartetem Maßstab zeigt die Resistenz der untersuchten Mikroben gegen das Antibiotikum in der verwendeten Konzentration an; suszeptible Mikroben zeigen kein Wachstum, wenn die Konzentration des Antibiotikums über der minimalen Hemmkonzentration (MHK) liegt.

Für die Identifizierung werden überwiegend Flugzeitmassenspektrometer mit einer Ionisierung durch matrixunterstützte Laserdesorption (MALDI) verwendet. MALDI hat jahrzehntelang unter dem Ruf gestanden, nicht für quantitative Analysen geeignet zu sein. Es hat sich aber des längeren herausgestellt, dass diese Aussage nicht stimmt, sondern nur den früher benutzten, sehr groben Präparationsverfahren für die Proben auf dem Probenträger zuzuschreiben war. Es kann gezeigt werden, dass sich beispielsweise eine Substanz mit MALDI bei sehr guter und gleichmäßiger Dünnschicht-Präparation und unter Zugabe von Referenzsubstanzen in entsprechend präzisen Dosierungen auf zwei Prozent genau mengenmäßig bestimmen lässt. Diese Genauigkeit ist hier bei weitem nicht erforderlich; die dazu aufzuwendende Mühe würde auch das Routineverfahren unnötig komplizieren. Aber eine quantitative Genauigkeit von etwa 20 Prozent lässt sich auch im Routinelabor ohne größere Schwierigkeiten erreichen. Da es sich bei den Mikrobenzuwächsen in jeder Verdoppelungszeit um einen Faktor zwei handelt, bei zwei Verdoppelungszeiten also um einen Faktor vier, ist MALDI dieser Aufgabe der Messung der Mikrobenzuwächse leicht gewachsen, selbst wenn in Anwesenheit des Antibiotikums ein langsameres Wachstum stattfinden sollte, oder wenn nicht alle gesammelten Mikrobenkolonien resistent sind.

Es wird daher in der Erfindung vorgeschlagen, vor der Messung der Massenspektren der Mikroben eine oder mehrere als quantitative Referenzen geeignete Substanzen in geeigneten, genau bekannten Mengen (bzw. Konzentrationen) hinzuzufügen, wofür hier der Begriff "dosiert" verwendet wird. Die Referenzsubstanzen können bereits dem Kultivierungsmedium zugefügt werden; dann ist es aber erforderlich, dass die Referenzsubstanz von den Mikroben möglichst immer in gleicher Menge aufgenommen und nicht durch Verdau zerstört werden. Es ist daher vorteilhaft, die Referenzsubstanzen erst nach der Abtötung der Mikroben dosiert hinzuzufügen, und zwar entweder den Proteinen in der Aufschlussflüssigkeit der Mikroben im Zentrifugierröhrchen oder aber der Matrixlösung, die auf die getrockneten Proteine auf dem MALDI-Probenträger aufgebracht wird. Diese Referenzsubstanzen erlauben es, das Wachstum der Mikroben mit und ohne Zugabe von Antibiotika gegeneinander quantitativ abzuschätzen und daraus die Resistenz oder Suszeptibilität zu bestimmen. Die Genauigkeit für die verwendeten Mengen und Konzentrationen sollte möglichst bei etwa 10 Prozent liegen, um eine Gesamtgenauigkeit des Verfahrens von etwa 20 Prozent zu erhalten. Die Referenzsubstanzen sollen durch eine hohe Protonenaffinität gut zu ionisieren sein, damit ihre Ionisierung nicht von den Proteinen der Mikroben unterdrückt werden kann, und nach Möglichkeit mehrere leicht erkennbare Peaks in den Massenspektren liefern. Es ist günstig, wenn die Referenzsubstanzen einen größeren Mengenbereich abdecken, so können beispielsweise drei Referenzsubstanzen in Verhältnissen von 100:10:1 oder 25:5:1 verwendet werden. Es hat sich als vorteilhaft erwiesen, wenn die Referenzsubstanz mit der höchsten Konzentration im Massenspektrum Referenzsignale erzeugt, die etwa die gleiche Höhe (Intensität) haben, wie sie die höchsten Mikrobensignale (in der Regel Proteinsignale))nach vollem, ungestörtem Wachstum zeigen.

Als Beispiel für eine gut verwendbare Substanz wird hier die Ribonuklease A angeführt. Sie hat ein Molekulargewicht von 13638 Dalton; ihre hohe Protonenaffinität bewirkt, dass im MALDI-Spektrum sowohl einfach, wie auch zweifach und sogar dreifach geladene Ionen der Ribonuklease A erscheinen. Die einfach geladenen RNase-A-Ionen erscheinen einem Gebiet des Massenspektrums, in dem sie in der Regel ohne Störungen durch andere Peaks gut erkennbar sind.

Als weitere geeignete Substanzen können hier beispielsweise andere Ribonukleasen angeführt werden. Aber auch andere Substanzklassen mit hoher Protonenaffinität können hier verwendet werden, beispielsweise Lysozyme. So hat Lysozym C eine Molekularmasse von 14,3 Kilodalton, bietet also ebenfalls einen Peak in einem wenig besetztem Bereich des Massenspektrums.

Das Verfahren hat sich als überraschend schnell erwiesen: Gefährliche Infektionen beruhen in der Regel auf schnell wachsenden Mikroben mit nur etwa 20 Minuten Verdopplungszeit. Da nur etwa zwei Verdopplungsgenerationen notwendig sind, um die Biomasse um einen Faktor vier wachsen zu lassen, kann für diese schnell wachsenden Mikroben deren Resistenz bereits nach einer Kultivierungsdauer von nur etwa 40 Minuten nachgewiesen werden; für langsamer wachsende Mikroben mit einer Verdoppelungszeit von 30 Minuten ist eine Stunde erforderlich. Rechnet man noch 20 Minuten für die Aufbereitung der Mikroben, der Präparation für die MALDI-Ionisierung und die Aufnahme der Massenspektren hinzu, so kann die Resistenz ein bis anderthalb Stunden nach deren Identifizierung feststehen. Es wirkt sich hier als Vorteil aus, dass die Mikroben vor der Resistenzbestimmung identifiziert wurden: Damit kann die benötigte Kultivierungsdauer durch die Kenntnis der Mikrobenart und ihrer Verdoppelungszeit optimal festgelegt werden.

Das Verfahren bietet außerdem Kontrollmöglichkeiten gegen Irrtümer beim Sammeln der Mikroben oder der Präparation der Proben: Die gewonnenen Massenspektren der Mikroben nach ihrer Kultivierung können noch einmal der Identifizierungsroutine für die Mikroben unterworfen werden und so die richtige Zuordnung bestätigen. Da dieses Verfahren der Identifizierung zeitintensiv ist, kann man es beschleunigen, indem man einfach die Ähnlichkeiten zwischen den Massenspektren nach der Kultivierung mit dem Massenspektrum, das für die Identifizierung verwendet wurde, bestimmt werden. Die Ähnlichkeitswerte geben Aufschluss darüber, ob für die Resistenzbestimmung die richtigen Massenspektren vorliegen.

Zwischen voller Resistenz der Mikroben und voller Suszeptibilität gibt es intermediäre Zwischenstufen; das Wachstum ist zwar beeinträchtigt, aber nicht voll gehemmt. Um die Stärke der Resistenz von Mikroben bestimmen oder zumindest abschätzen zu können, können die tatsächlichen Hemmkonzentrationen der Antibiotika gemessen werden. Die MHK-Werte der Antibiotika (minimale Hemmkonzentrationen für voll suszeptible Mikroben) sind weitgehend bekannt; die aktuellen Hemmkonzentrationen steigen aber mit der Stärke der Resistenz an. Für die Messung der aktuellen Hemmkonzentrationen können Kulturen mit Zugabe eines Antibiotikums in verschiedenen Konzentrationsabstufungen verwendet werden, die beispielsweise den Konzentration 1*MHK, 10*MHK und 100*MHK der bekannten MHK-Werte entsprechen können. Erfahrungsgemäß lassen sich mit dem oben geschilderten Verfahren die Hemmung des Mikrobenwachstums bei einer Konzentration von 1*MHK nur dann feststellen, wenn die Mikroben voll suszeptibel sind. Bei Vorliegen einer schwachen Resistenz werden sie erst bei einer Konzentration von 10*MHK gehemmt; während für eine sehr starke Resistenz selbst bei einer Konzentration von 100*MHK noch ein Wachstum festzustellen ist. Die Wirkung kann an den Werten der Mikrobenzuwächse abgelesen werden. Es ergibt sich also für intermediäre Resistenzen verschiedenes Wachstum bei verschiedenen Konzentrationen des Antibiotikums.

Es kann aber auch der Fall vorliegen, dass von den eingesammelten Kolonien nur ein Teil, beispielsweise die Hälfte, resistent und die anderen suszeptibel sind. Wir sprechen hier von einer "Mischresistenz". Dann wird auch bei starker Resistenz anscheinend weniger Wachstum festgestellt, aber nur, weil sich in der Kultur eine geringere Ausgangsmenge an resistenten Mikroben befand. Wird hier der Test mit verschiedenen Konzentrationen des Antibiotikums durchgeführt, so ist bei allen Konzentration eine im gleichen Prozentsatz geringer gewachsene Proteinmenge gegenüber der Kultur ohne Antibiotikum festzustellen.

Wird das Verfahren ohne Konzentrationsabstufungen durchgeführt, so hat sich eine Konzentration 10*MHK als besonders geeignet erwiesen.

Für die Prüfung der Resistenz gegenüber mehreren Antibiotika können mehrere Kulturen mit mehreren Antibiotika angesetzt werden, erforderlichenfalls sogar mit jeweils verschiedenen Konzentrationsabstufungen der Antibiotika. Der zusätzliche Zeitaufwand für die Präparation der Mikroben aus mehreren Kulturen fällt gegenüber dem Zeitaufwand der Kultivierung kaum ins Gewicht.

Für eine schnelle Prüfung auf multiresistente Keime (Beispiel: MRSA, methicillinresistenter Staphylokokkus aureus;) können die Medien auch mit einem Gemisch aus mehreren Arten von Antibiotika versehen werden. Wachsen die Mikroben in diesem Gemisch, so sind sie multiresistent. Bei dieser Schnellprüfung kann eine zu untersuchende Probe, wie z.B. der Abstrich der Nasenschleimhaut, auch ein Mikrobengemisch aufweisen und es kann auf eine vorhergehende Identifizierung der Mikroben in der Probe verzichtet werden. Eine Identifizierung der im Medium mit Antibiotikum gewachsenen und damit als resistent ermittelten Mikroben ist mittels des aufgenommenen Massenspektrums möglich.

In einem bevorzugten Verfahren werden die Intensitäten aller Massenpeaks in einem ausgewählten Spektrenausschnitt, beispielsweise von 4000 bis 10000 Dalton, summiert und durch die Intensität des Peaks der einfach geladenen Referenzionen geteilt: es ergibt sich dabei ein Quotient *q*. Man kann das Routineverfahren für die Resistenzbestimmung so ausfeilen, dass Werte dieses Quotienten *q* unter 200 suszeptible Peaks ohne Wachstum ergeben (viele der hier vorhandenen Peaks sind Rauschen); während Werte des Quotienten *q* über 200 Resistenz anzeigen. Bevorzugter ist ein Auswertungsverfahren, das jeweils die Quotienten *Q = q_{cum}*/*qₛᵢₙₑ* für Mikroben in Medien mit (*q_{cum}*) und ohne Antibiotika (*qₛᵢₙₑ*) bildet. Liegt dieser Quotient näherungsweise bei eins (0,8 < Q < 1,3), so liegt eine Resistenz der Mikroben vor; für suszeptible Keime liegt Q etwa bei 0,25 (0,1 < Q < 0,4), wenn die Kultivierungszeit etwa zwei Verdoppelungszeiten beträgt.

Für Routinelaboratorien mit größerem Anfall an Mikrobenproben, die sowohl identifiziert wie auch auf ihre Resistenz geprüft werden müssen, ist es wertvoll, zumindest Teile der Verfahrensschritte automatisieren zu können. Eine vollständige Automatisierung des gesamten Verfahrens ist zurzeit noch nicht möglich; jedoch gibt es bereits eine Reihe von Apparaturen auf dem Markt oder in marktnaher Entwicklung, die zumindest einige Verfahrensschritte automatisch oder halbautomatisch erledigen können. So gibt es ein Gerät, das entweder unter visueller Kontrolle oder aber durch Bildauswertung Mikrobenkolonien von Agar-Platten ernten kann und die Mikroben für eine Identifizierung auf die Probenträgerplatte aufbringen kann. Dieses Gerät kann leicht weiter entwickelt werden, um auch Mikroben für die Resistenzbestimmung zu ernten. Geräte zum Aufschluss der Mikroben auf der Probenträgerplatte und zur Präparation mit Matrixlösung sind ebenfalls denkbar. Es sind Pipettierstationen erhältlich, die in geeigneten Mikrotitrierplatten oder Serien von Zentrifugenröhrchen den Aufschluss der Zentrifugen-Pellets vornehmen können. Die Kultivierung kann jeweils in Zentrifugenröhrchen (beispielsweise Eppendorf-Röhrchen) oder in Filterplatten (beispielsweise Acropep 96-well Filterplatten) vorgenommen werden. Es sind IVD-zertifizierte Verfahren für MALDI-Massenspektrometer auf dem Markt, die mit Probenträgern arbeiten, die 48, 96 oder 384 Proben aufnehmen können.

Für eine Ionisierung durch matrixunterstützte Laserdesorption (MALDI) ist entweder eine Probenträgerplatte, auf der die Matrixsubstanz in Dünnschicht bereits fertig präpariert ist, oder die Anfertigung einer Matrixlösung erforderlich. Industriell vertriebene Matrixsubstanzen haben häufig den Nachteil, dass sie sich ohne die Anwendung von Ultraschall nur schwer lösen lassen. Es sind daher Fläschchen mit gereinigten und gefriergetrockneten Matrixsubstanzen in genau dosierten Mengen erhältlich, in denen sich die Matrixsubstanz bei Zugabe der Lösungsmittel sofort auflöst und die Lösung in richtiger Konzentration sofort verwendungsfähig ist. Im Sinne dieser Erfindung kann den Matrixsubstanzen dieser Produkte wohldosiert mindestens eine Referenzsubstanz für die Quantifizierung des Proteinzuwachses beigegeben werden. Die Matrixlösung kann in dem Gerät zur Präparation der MALDI-Proben gut dosiert und berührungslos auf die getrockneten Zellbestandteile der Mikroben, vor allem Proteine, aufgebracht werden. Auch die Probenträgerplatten mit Matrixdünnschichten, die bereits als Produkte vertrieben werden, können Referenzsubstanzen in dosierten Mengen enthalten. Die Dünnschichten sind jeweils auf kleine, voneinander gut getrennte Probenbereiche von je etwa zwei Millimeter Durchmesser aufgebracht.

Der Ablauf eines bevorzugten Verfahrens zur Bestimmung von Resistenzen ist im Diagramm der Abbildung 1 als Beispiel wiedergegeben. Das Verfahren ist hier mit einer Kultivierung der Mikroben auf einem Agar dargestellt (101). Die Mikroben einer Kolonie werden geerntet (102), aufgeschlossen und zu einer MALDI-Probe verarbeitet (103). Die Aufnahme eines Massenspektrums (104) führt zur Identifizierung der Mikrobe durch Vergleich ihres Massenspektrums mit Referenzspektren (105). Ab Ernten einer Kolonie bis zur Identifizierung dauert es in einem Routine-Labor nur 10 bis 30 Minuten, je nach der Anzahl der parallel zu identifizierenden Mikrobenproben. Für die Bestimmung der Resistenz können zeitgleich mehrere weitere Kolonien der gleichen Mikroben geerntet werden (106). Diese werden gemischt und für die verschiedenartigen Kulturen aufgeteilt (107). Im Beispiel dieses Diagramms werden drei Kulturen angesetzt: eine Kultur in einem Medium ohne Antibiotikum (108), und zwei Kulturen mit den Antibiotika Ab1 (109) und Ab2 (110). Selbstredend können weitere Kulturen mit weiteren Antibiotika, und, wenn auch die Stärke der Resistenz bestimmt werden soll, Kulturen mit verschiedenen Konzentrationen der Antibiotika angesetzt werden. Alle Kulturen werden bereits mit optimaler Temperatur angesetzt, um die Mikroben nicht unter Schock zu setzen und keine Zeitzögerung durch das Erwärmen zu haben. Die Dauer der Kultivierung richtet sich nach der Verdoppelungszeit (Generationsdauer) der Mikroben, die durch die Identifizierung der Mikroben bekannt ist: Die Kultivierung braucht nur zwei bis drei Verdoppelungszeiten zu dauern. Für schnell wachsende Mikroben genügen etwa 40 Minuten. Die Mikroben aus den verschiedenen Kulturen werden dann unter Zugabe der Referenzsubstanzen zu MALDI-Proben verarbeitet, und es werden Massenspektren aufgenommen (111). Aus dem Massenspektrum der Mikroben, die ohne Antibiotikum kultiviert wurden, wird der Quotient *q*ₛᵢₙₑ gebildet, indem die Intensitäten aller Peaks im Massenbereich von beispielsweise 4000 bis 10000 Dalton addiert und durch die Intensität des Referenzpeaks dividiert werden. Aus den Massenspektren der Mikroben, die mit dem jeweiligen Antibiotikum *n* kultiviert wurden, werden die entsprechenden Quotienten *q*_{cum,n} gebildet (112). Die Quotienten *Q*ₙ = *q*_{cum,n}/*q*ₛᵢₙₑ zeigen, wie oben bereits angegeben, die jeweilige Resistenz an.

Es wurden die Verfahren bisher mit einer Ionisierung durch MALDI in einem MALDI-Flugzeitmassenspektrometer durchgeführt. MALDI hat den großen Vorteil, ganz überwiegend nur einfach geladene Molekül-Ionen zu bilden. Damit werden die Massenspektren trotz der 50 bis 100 Peaks, die im bevorzugten Massenbereich von 3000 bis 15000 Dalton erscheinen, nicht überladen, und es können Ähnlichkeiten relativ einfach erkannt werden. Das heißt aber nicht, dass nicht auch andere Arten der Ionisierung eingesetzt werden könnten. Die sprüh-basierten Verfahren wie ESI (Elektrosprüh-Ionisierung) oder DESI (direkte Oberflächen-Ionisierung fester Proben durch Elektrosprühen) bilden vielfach geladene Ionen, die die Massenspektren leicht überladen, lassen sich aber mit Separationsverfahren wie Flüssigkeitschromatographie (HPLC) oder Kapillarelektrophorese (CE) koppeln, so dass durch die Trennung der Substanzen wieder einfacher aufgebaute Massenspektren erhalten werden können.

Es gibt jedoch auch andere Ionisierungsverfahren, die fast nur einfach geladene Ionen erzeugen, beispielsweise die chemische Ionisierung (CI). Die chemische Ionisierung kann in Verbindung mit Neutral-Sprühverfahren, aber auch mit Laser-Ablation von festen Proben eingesetzt und in Verbindung mit einem OTOF-MS (Flugzeitmassenspektrometer mit orthogonalem Ioneneinschuss) verwendet werden. Die so erhaltenen Massenspektren bieten bei hoher Empfindlichkeit höchste Massenauflösung (siehe beispielsweise J. Franzen und K. Michelmann, DE 10 2005 044 307 B4).

Selbstredend können auch andere Arten von Massenspektrometern eingesetzt werden, wenn sie den bevorzugten Massenbereich von 3000 bis 15000 Dalton für die Messung der Massenspektren bieten.

## Patentansprüche

1. Verfahren zur massenspektrometrischen Bestimmung der Resistenz von Mikroben, in dem die Mikroben in einem Medium mit einem Antibiotikum kultiviert werden und ein Massenspektrum MS_{cum} der Mikroben nach deren Kultivierung aufgenommen wird, **dadurch gekennzeichnet, dass** der während der Kultivierung gegebenenfalls stattfindende Zuwachs an Mikroben anhand einer dosiert zugegebenen, im Massenspektrum MS_{cum} mitgemessenen Referenzsubstanz bestimmt wird, wobei ein Zuwachs an Mikroben die Resistenz gegen das Antibiotikum anzeigt.

2. Verfahren nach Anspruch 1, **dadurch gekennzeichnet, dass** die Mikrobenmenge nach der Kultivierung aus dem Massenspektrum MS_{cum} ermittelt wird und dass die Mikroben als resistent ausgewiesen werden, wenn die Mikrobenmenge größer als ein vorgegebener Grenzwert ist.

3. Verfahren nach einem der Ansprüche 1 oder 2, **dadurch gekennzeichnet, dass** die Mikroben zusätzlich in dem Medium ohne das Antibiotikum kultiviert werden, dass ein Massenspektrum MSₛᵢₙₑ der Mikroben nach deren Kultivierung ohne Antibiotikum aufgenommen wird, in dem die dosiert zugegebene Referenzsubstanz mitgemessen wird, und dass die Mikroben als resistent ausgewiesen werden, wenn sich die aus den Massenspektren MS_{cum} und MSₛᵢₙₑ ermittelten Mikrobenmengen relativ oder absolut um weniger als einen vorgegebenen Grenzwert unterscheiden.

4. Verfahren nach einem der Ansprüche 1 bis 3, **dadurch gekennzeichnet, dass** vor der Kultivierung zusätzlich ein Massenspektrum MS₀ der Mikroben aufgenommen wird, in dem die dosiert zugegebene Referenzsubstanz mitgemessen wird, und dass die Mikroben als resistent ausgewiesen werden, wenn die aus dem Massenspektrum MS_{cum} ermittelte Mikrobenmenge signifikant größer als die aus dem Massenspektrum MS₀ ermittelte Mikrobenmenge ist.

5. Verfahren nach einem der Ansprüche 2 bis 4, **dadurch gekennzeichnet, dass** die Mikrobenmenge als Quotient aus einem Massenspektrum ermittelt wird, wobei
- der Quotient aus der Intensität eines Mikrobensignals und der Intensität eines Referenzsignals oder den summierten Intensitäten mehrerer Referenzsignale gebildet wird,
- der Quotient aus den summierten Intensitäten mehrerer Mikrobensignale und der Intensität eines Referenzsignals oder den summierten Intensitäten mehrere Referenzsignale gebildet wird, oder
- der Quotient aus den summierten Intensitäten aller Signale eines Bereiches oder des ganzen Massenspektrums und der Intensität eines Referenzsignals oder den summierten Intensitäten mehrerer Referenzsignale gebildet wird.

6. Verfahren nach einem der Ansprüche 1 bis 5, **dadurch gekennzeichnet, dass** die Referenzsubstanz nach einer Kultivierung der Mikroben dosiert zugegeben wird.

7. Verfahren nach einem der Ansprüche 1 bis 5, **dadurch gekennzeichnet, dass** die Mikroben zu einer massenspektrometrischen Probe präpariert werden und die Referenzsubstanz bei der Präparation dosiert zugegeben wird.

8. Verfahren nach Anspruch 7, **dadurch gekennzeichnet, dass** die Massenspektren mittels Ionisierung durch matrixunterstützte Laserdesorption (MALDI) aufgenommen werden und die Referenzsubstanz bei der Präparation der MALDI-Proben auf einem Probenträger zugegeben wird.

9. Verfahren nach einem der Ansprüche 1 bis 8, **dadurch gekennzeichnet, dass** die Mikroben gleichzeitig in mehreren Kulturen mit jeweils einem anderen Antibiotikum kultiviert werden und für jede Kultur ein Massenspektrum der Mikroben der Referenzsubstanz aufgenommen wird.

10. Verfahren nach einem der Ansprüche 1 bis 9, **dadurch gekennzeichnet, dass** die Mikroben gleichzeitig in mehreren Kulturen mit jeweils einer anderen Konzentration des Antibiotikums kultiviert werden und für jede Kultur ein Massenspektrum der Mikroben und der Referenzsubstanz aufgenommen wird.

11. Verfahren nach einem der Ansprüche 1 bis 10, **dadurch gekennzeichnet, dass** die Protonenaffinität der Referenzsubstanz größer als die Protonenaffinität der Mehrzahl der Proteine der Mikroben ist.

12. Verfahren nach Anspruch 11, **dadurch gekennzeichnet, dass** die Referenzsubstanz eine Ribonuklease oder ein Lysozym ist.

13. Verfahren nach einem der Ansprüche 1 bis 12, **dadurch gekennzeichnet, dass** vor oder während der Präparation einer massenspektrometrischen Probe die Proteine der Mikroben aufkonzentriert werden.

14. Verfahren nach einem der Ansprüche 1 bis 13, **dadurch gekennzeichnet, dass** ein Gemisch von Referenzsubstanzen dosiert zugegeben und in den Massenspektren der Mikroben mitgemessen wird.

15. Verfahren nach einem der Ansprüche 1 bis 14, **dadurch gekennzeichnet, dass** die Mikroben vor der Bestimmung der Resistenz taxonomisch identifiziert werden und dass die Referenzsubstanz, die Grenzwerte zur Bestimmung der Resistenz, das Medium und/oder die Kultivierungsbedingungen aufgrund der taxonomischen Einordnung ausgewählt werden.

## Claims

1. Method for the mass-spectrometric determination of the resistance of microbes, in which the microbes are cultured in a medium containing an antibiotic, and a mass spectrum MS_{cum} of the microbes is acquired after they have been cultured, wherein any growth of microbes taking place during the culture is determined with the aid of a dosed addition of a reference substance, which is also measured in the mass spectrum MS_{cum}, and microbial growth indicates the resistance to the antibiotic.

2. Method according to Claim 1, wherein the quantity of microbes is determined after culturing from the mass spectrum MS_{cum}, and the microbes are identified as being resistant if the quantity of microbes exceeds a specified limit value.

3. Method according to Claim 1 or 2, wherein the microbes are additionally cultured in the medium without the antibiotic; a mass spectrum MSₛᵢₙₑ of the microbes is acquired after culturing without the antibiotic, wherein the reference substance added in a dosed amount is also measured in the mass spectrum; and the microbes are identified as resistant if the quantities of microbes derived from the mass spectra MS_{cum} and MSₛᵢₙₑ differ in relative or absolute terms by less than a specified limit value.

4. Method according to one of the Claims 1 to 3, wherein before the culture, a mass spectrum MS₀ of the microbes is also acquired, in which the reference substance added in a dosed amount is also measured, and wherein the microbes are identified as being resistant if the quantity of microbes determined from the mass spectrum MS_{cum} significantly exceeds the quantity of microbes which was determined from the mass spectrum MS₀.

5. Method according to one of the Claims 2 to 4, wherein the quantity of microbes is determined as a quotient from a mass spectrum, where
- the quotient is formed from the intensity of a microbe signal and the intensity of a reference signal or the summed intensities of several reference signals,
- the quotient is formed from the summed intensities of several microbe signals and the intensity of a reference signal or the summed intensities of several reference signals, or
- the quotient is formed from the summed intensities of all the signals within one region or within the whole mass spectrum and the intensity of a reference signal or the summed intensities of several reference signals.

6. Method according to one of the Claims 1 to 5, wherein the dosed reference substance is added after the microbes have been cultured.

7. Method according to one of the Claims 1 to 5, wherein the microbes are prepared in the form of a mass-spectrometric sample and the dosed reference substance is added during the preparation.

8. Method according to Claim 7, wherein the mass spectra are acquired by means of ionization by matrix-assisted laser desorption (MALDI), and the reference substance is added during preparation of the MALDI samples on a sample support.

9. Method according to one of the Claims 1 to 8, wherein the microbes are cultured simultaneously in several cultures, each having a different antibiotic, and a mass spectrum of the microbes and of the reference substance is acquired for each culture.

10. Method according to one of the Claims 1 to 9, wherein the microbes are simultaneously cultured in several cultures, each having a different concentration of the antibiotic, and a mass spectrum of the microbes and of the reference substance is acquired for each culture.

11. Method according to one of the Claims 1 to 10, wherein the proton affinity of the reference substance is greater than the proton affinity of most of the proteins of the microbes.

12. Method according to Claim 11, wherein the reference substance is a ribonuclease or a lysozyme.

13. Method according to one of the Claims 1 to 12, wherein the proteins of the microbes are concentrated before or during the preparation of a mass-spectrometric sample.

14. Method according to one of the Claims 1 to 13, wherein a dosed mixture of reference substances is added and also measured in the mass spectra of the microbes.

15. Method according to one of the Claims 1 to 14, wherein the microbes are identified taxonomically before their resistance is determined, and the reference substance, the limit values for determining the resistance, the medium and/or the culture conditions are selected on the basis of the taxonomic classification.

## Revendications

1. Méthode de la spectrométrie de masse utilisée pour déterminer la résistance de microbes et consistant à cultiver avec un antibiotique les microbes dans un milieu et d'enregistrer un spectre de masse MS_{cum} des microbes une fois la culture terminée, **caractérisée en ce que** la croissance des microbes pouvant se produire pendant la culture est déterminée au moyen d'une substance de référence ajoutée sous forme dosée et mesurée dans le spectre de masse MS_{cum} et qu'une croissance des microbes indique que les microbes sont résistants à l'antibiotique.

2. Méthode selon la revendication 1, **caractérisée en ce que** la quantité de microbes est calculée une fois la culture terminée à partir du spectre de masse MS_{cum} et que les microbes sont dits résistants si la quantité de microbes est supérieure à une valeur seuil spécifique.

3. Méthode selon l'une des revendications 1 ou 2, **caractérisée en ce que** les microbes sont en plus cultivés sans l'antibiotique dans le milieu, qu'un spectre de masse MSₛᵢₙₑ des microbes, dans lequel est également mesurée la substance de référence ajoutée sous forme dosée, est enregistré une fois la culture des microbes sans antibiotique terminée et que les microbes sont dits résistants si les quantités de microbes calculées à partir des spectres de masse MS_{cum} et MSₛᵢₙₑ se différencient de manière relative ou absolue l'une par rapport à l'autre d'une valeur inférieure à une valeur seuil spécifique.

4. Méthode selon l'une des revendications 1 à 3, **caractérisée en ce qu'**un spectre de masse MS₀ des microbes, dans lequel est également mesurée la substance de référence ajoutée sous forme dosée, est en plus enregistré avant le début de la culture et que les microbes sont dits résistants si la quantité de microbes calculée à partir du spectre de masse MS_{cum} est nettement supérieure à la quantité de microbes calculée à partir du spectre de masse MS₀.

5. Méthode selon l'une des revendications 2 à 4, **caractérisée en ce que** la quantité de microbes est calculée comme quotient à partir d'un spectre de masse, étant entendu que :
- Le quotient se compose de l'intensité d'un signal microbien et de l'intensité d'un signal de référence, ou de la somme des intensités de plusieurs signaux de référence ;
- Le quotient se compose de la somme des intensités de plusieurs signaux microbiens et de l'intensité d'un signal de référence, ou de la somme des intensités de plusieurs signaux de référence ; ou
- Le quotient se compose de la somme des intensités de tous les signaux d'une zone ou de l'ensemble du spectre de masse et de l'intensité d'un signal de référence, ou de la somme des intensités de plusieurs signaux de référence.

6. Méthode selon l'une des revendications 1 à 5, **caractérisée en ce que** la substance de référence est ajoutée sous forme dosée une fois la culture des microbes terminée.

7. Méthode selon l'une des revendications 1 à 5, **caractérisée en ce que** les microbes sont préparés en un échantillon de spectrométrie de masse et que la substance de référence est ajoutée sous forme dosée lors de la préparation.

8. Méthode selon la revendication 7, **caractérisée en ce que** les spectres de masse sont enregistrés au moyen d'une désorption-ionisation laser assistée par matrice (MALDI) et que la substance de référence est ajoutée lors de la préparation des échantillons MALDI sur un porte-échantillon.

9. Méthode selon l'une des revendications 1 à 8, **caractérisée en ce que** les microbes sont cultivés simultanément dans plusieurs cultures contenant chacune un antibiotique différent et qu'un spectre de masse des microbes et de la substance de référence est enregistré pour chaque culture.

10. Méthode selon l'une des revendications 1 à 9, **caractérisée en ce que** les microbes sont cultivés simultanément dans plusieurs cultures pour chacune desquelles la concentration de l'antibiotique est différente, et qu'un spectre de masse des microbes et de la substance de référence est enregistré pour chaque culture.

11. Méthode selon l'une des revendications 1 à 10, **caractérisée en ce que** l'affinité protonique de la substance de référence est supérieure à l'affinité protonique de la majorité des protéines microbiennes.

12. Méthode selon la revendication 11, **caractérisée en ce que** la substance de référence est une ribonucléase ou un lysozyme.

13. Méthode selon l'une des revendications 1 à 12, **caractérisée en ce que** la quantité des protéines microbiennes est augmentée par concentration avant ou pendant la préparation d'un échantillon par spectrométrie de masse.

14. Méthode selon l'une des revendications 1 à 13, **caractérisée en ce qu'**un mélange de substances de référence est ajouté sous forme dosée et mesuré dans les spectres de masse des microbes.

15. Méthode selon l'une des revendications 1 à 14, **caractérisée en ce qu'**une identification taxonomique des microbes est effectuée avant la détermination de la résistance et que la substance de référence, les valeurs seuils permettant de déterminer la résistance, le milieu et/ou les conditions de culture sont choisis en fonction de la classification taxonomique.
